(19) Europäisches Patentamt / European Patent Office / Office européen des brevets

(11) **EP 3 982 869 B1**

(12) **EUROPÄISCHE PATENTSCHRIFT**

(45) Veröffentlichungstag und Bekanntmachung des Hinweises auf die Patenterteilung:
**11.03.2026  Patentblatt 2026/11**

(21) Anmeldenummer: **20733556.3**

(22) Anmeldetag: **12.06.2020**

(51) Internationale Patentklassifikation (IPC):
**A61C 8/02** *(2006.01)*       **A61F 2/28** *(2006.01)*
**A61C 8/00** *(2006.01)*

(52) Gemeinsame Patentklassifikation (CPC):
**A61C 8/0006; A61C 8/0012; A61C 8/0043; A61F 2/2846;** A61F 2002/2835; A61F 2002/285; A61F 2002/2889

(86) Internationale Anmeldenummer:
**PCT/EP2020/066359**

(87) Internationale Veröffentlichungsnummer:
**WO 2020/249765 (17.12.2020 Gazette 2020/51)**

(54) **ZAHNTECHNISCHES IMPLANTAT UND SET**

DENTAL IMPLANT AND SET

IMPLANT DENTAIRE ET ENSEMBLE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **14.06.2019  DE 102019116244**
**05.08.2019  DE 102019121083**

(43) Veröffentlichungstag der Anmeldung:
**20.04.2022  Patentblatt 2022/16**

(73) Patentinhaber:
• **Randelzhofer, Peter**
  **80804 München (DE)**
• **Stur, Stephan**
  **80804 München (DE)**

(72) Erfinder:
• **Randelzhofer, Peter**
  **80804 München (DE)**
• **Stur, Stephan**
  **80804 München (DE)**

(74) Vertreter: **Bobbert & Partner**
**Patentanwälte PartmbB**
**Postfach 1252**
**85422 Erding (DE)**

(56) Entgegenhaltungen:
DE-A1- 102016 000 236        DE-A1- 102016 000 236
KR-B1- 101 231 581           KR-B1- 101 231 581
US-A- 5 769 637              US-A- 5 769 637
US-A1- 2005 192 675          US-A1- 2005 192 675

**Beschreibung**

Die vorliegende Erfindung betrifft ein zahntechnisches Implantat gemäß Anspruch 1 und ein Set gemäß Anspruch 10 .

Als Zahnimplantat wird üblicherweise ein in den Kieferknochen einsetzbares Implantat bezeichnet, das auch als künstliche Zahnwurzel bezeichnet werden kann. Das Zahnimplantat ist somit eine Basis für einen hierauf aufbauenden Zahnersatz. In das Zahnimplantat wird in der Regel zunächst ein Verbindungsteil, das als *abutment* bezeichnet wird, eingeschraubt und anschließend die sichtbare Zahnkrone als prothetische Versorgung auf das Verbindungsteil aufgesetzt.

Für das Einsetzen des Zahnimplantats in den Kieferknochen ist für eine dauerhafte Verankerung des Zahnimplantats ein stabiler und ausreichend großer Kieferknochenbereich notwendig. Dies ist jedoch oftmals nicht gegeben, beispielsweise weil sich der Kieferknochen nach einer Extraktion eines natürlichen Zahns und einer Wundheilphase zurückgebildet hat, oder weil der Kieferknochen patientenindividuell zart ausgebildet ist. Die Rede ist hierbei oft von einem Knochendefekt oder einer Knochendefektstelle. In solchen Fällen kann es notwendig sein, vor dem Implantieren des Zahnimplantats den Kieferknochen "aufzubauen". Zum Aufbau kann ein Knochenaufbaumaterial aus einem synthetischen Knochenersatzmaterial, beispielsweise aus Hydroxylapatit, und/oder aus einem körpereigenen Knochenersatzmaterial verwendet werden.

Während der Knochenaufbauphase sollte der Bereich des Kieferknochens, welcher das Knochenaufbaumaterial aufgetragen bekommen hat, gegen ein unerwünschtes Einwachsen von umgebendem Schleimhautgewebe oder anderem Weichgewebe abgegrenzt werden, damit das Knochenaufbaumaterial ungestört, vom Kieferknochen ausgehend, knöchern durchwachsen werden kann. Zu diesem Zweck wird das Knochenaufbaumaterial regelmäßig mit einer sogenannten Abdeckmembran bedeckt, die ein Durchwachsen des Knochenaufbaumaterials mit einem Nicht-Knochenmaterial, beispielsweise umgebendem Schleimhautgewebe, verhindern soll.

Die Abdeckmembran wird regelmäßig aus einem Polymer hergestellt, beispielsweise aus Polytetrafluorethylen, abgekürzt PTFE. Die Abdeckmembran kann als Abdeckfolie bezeichnet werden. Die temporäre Befestigung der Abdeckmembran am Kieferknochen kann mittels geeigneter Befestigungsmittel, beispielsweise mittels Nägeln, erfolgen.

Zum möglichst ungestörten knöchernen Durchwachsen des Knochenaufbaumaterials ist es vorteilhaft, wenn die Abdeckmembran einen Hohlraum bzw. ausreichend Platz für das Knochenwachstum bereitstellt. Damit kann im Innern des Hohlraums ein Knochenaufbau bzw. eine Knochenregeneration möglichst ohne äußere Störungen, wie beispielsweise einem mechanischen Druck durch das umliegende Gewebe, erfolgen. Zu diesem Zweck kann die Abdeckmembran beispielsweise steif und/ oder formstabil ausgestaltet sein.

Aus der DE 10 2016 000 236 A1 sind ein Verfahren zur Herstellung eines Aufsatzes einer Abdeckvorrichtung für eine Knochendefektstelle und eine Vorrichtung zur Abdeckung und/oder Rekonstruktion einer Knochendefektstelle bekannt.

Aus der US 5,769,637 A ist eine Zahnprothese bekannt.

Aus der KR 101 231 581 B1 ist ein weiteres zahntechnisches Implantat bekannt.

In der US 2005/192675 A1 ist eine Einrichtung zur Stimulation des Knochenwachstums offenbart.

Eine Aufgabe der vorliegenden Erfindung kann es sein, ein zahntechnisches Implantat (alternative Bezeichnung: *"Bone Shield",* die Begriffe "zahntechnisches Implantat" und "Bone Shield" sind daher hierin austauschbar) anzugeben, welches zum Abstützen, insbesondere mittels einer Stützstruktur wie einem Stützelement, einer Abdeckmembran vorgesehen und ausgestaltet ist. Weiterhin ist eine Aufgabe der vorliegenden Erfindung, ein Set mit einem zahntechnischen Implantat anzugeben.

Die erfindungsgemäße Aufgabe wird durch das zahntechnische Implantat mit den Merkmalen des Anspruchs 1 und das Set mit den Merkmalen des Anspruchs 10 gelöst.

Hierzu schlägt die vorliegende Erfindung ein zahntechnisches Implantat mit den Merkmalen des Anspruchs 1 vor.

Das erfindungsgemäße zahntechnische Implantat weist wenigstens ein Verbindungselement und wenigstens ein erstes Stützelement auf. Das Verbindungselement umfasst einen ersten Endabschnitt auf. Es weist ferner einen zweiten Endabschnitt zum Verbinden des Implantats in oder mit einer Implantationsstelle eines Kieferknochens. Das erste Stützelement ist zum Tragen oder Abstützen eines Deckelements nach Implantation des erfindungsgemäßen Implantats an der Implantationsstelle im Kieferknochen ausgestaltet und vorgesehen. Das Deckelement ist ausgestaltet und vorgesehen, um zumindest einen Teil oder Abschnitt der Augmentationsstelle, der Implantationsstelle, der Implantationsregion oder eines benachbarten Bereichs zu bedecken.

Der erste Endabschnitt ist als ein erster Verbindungsabschnitt ausgestaltet oder weist einen solchen auf.

Das erste Stützelement weist einen zweiten Verbindungsabschnitt auf.

Der erste Verbindungsabschnitt und der zweite Verbindungsabschnitt sind ausgestaltet, um untereinander oder miteinander verbindbar zu sein.

Der zweite Endabschnitt ist als ein Implantationsabschnitt zum vorübergehenden Implantieren des Verbindungselements an oder in der Implantationsstelle des Kieferknochens ausgestaltet oder weist einen solchen auf.

Ferner schlägt die vorliegende Erfindung ein Set vor, welches ein erfindungsgemäßes zahntechnisches Implantat umfasst. Das Set weist ferner wenigstens ein Zahnimplantat zum dauerhaften Verbleib an der Implantationsstelle, -region oder Augmentationsstelle, vorzugsweise an der zuvor das zahntechnische Implantat vorübergehend implantiert war, auf, und/oder es weist ein weiteres Stützelement auf, das eines, mehrere oder alle Merkmale des hierin offenbarten ersten Stützelements haben kann, und/oder es weist ferner ein Werkzeug zum Einbringen des Verbindungselements in den Knochen auf. Das erste Stützelement und das optionale zweite Stützelement unterscheiden sich in wenigstens einem geometrischen Merkmal. Das unterscheidende geometrische Merkmal kann die Form, eine Abmessung, die Fläche und/ oder die Anordnung von Stützstrukturelementen umfassen.

Das hierin offenbarte, nicht die Erfindung darstellende Werkzeug dient dem Einbringen eines Verbindungselements eines erfindungsgemäßen zahntechnischen Implantats. Das Werkzeug weist einen Spaltabschnitt zum Aufnehmen eines Endbereichs des Verbindungselements auf.

Erfindungsgemäße Ausführungsformen können eines oder mehrere der oben und/oder im Folgenden genannten Merkmale in beliebiger Kombination aufweisen, sofern für den Fachmann die konkrete Ausführungsform nicht als technisch unmöglich erkennbar ist.

Bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen und soll eine erfindungsgemäße Ausführungsform erläutern.

Wann immer hierin Zahlenworte genannt werden, so versteht der Fachmann diese als Angabe einer zahlenmäßig unteren Grenze. Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

Vorteilhafte Weiterentwicklungen der vorliegenden Erfindung sind jeweils Gegenstand von Unteransprüchen und Ausführungsformen.

Wenn hierin von einer Ausführungsform die Rede ist, so stellt diese eine erfindungsgemäße, beispielhafte Ausführungsform dar.

Das zahntechnische Implantat ist vorzugsweise kein Zahnimplantat, das zum dauerhaften Verbleib oder zum Ersetzen eines Zahns gedacht und/oder geeignet ist. Es ist vorzugsweise nicht vorgesehen, um eine Zahnkrone zu tragen.

Ein Zahnimplantat kann als künstliche Zahnwurzel, als Aufnahme für die Zahnkrone, und/oder als Schraube bezeichnet werden. Ein Zahnimplantat kann eine Basis für einen hierauf aufbauenden Zahnersatz sein. In das Zahnimplantat kann ein Verbindungsteil, das oft als *abutment* bezeichnet wird, eingeschraubt werden. Anschließend kann eine sichtbare Zahnkrone als prothetische Versorgung auf das Verbindungsteil aufgesetzt werden.

In einigen Ausführungsformen ist das zahntechnische Implantat nicht zu seinem Befestigen und/oder zum Verbinden an oder mit einem Zahnimplantat ausgestaltet und/oder dafür vorbereitet.

In einigen Ausführungsformen ist das zahntechnische Implantat zum Abstützen einer Abdeckmembran vorbereitet und/oder ausgestaltet. Die Abdeckmembran kann temporär eine Knochendefektstelle mit einem Knochenaufbaumaterial abdecken, um einen Knochenaufbau am Kieferknochen für ein späteres Implantieren eines Zahnimplantats vorzubereiten. Mit der Abdeckmembran soll ein ungehinderter Knochenaufbau unterstützt und ein unerwünschtes Einwachsen von umgebendem Weichgewebe verringert oder verhindert bzw. unterbunden werden. Die Abdeckmembran kann während dieser Knochenaufbauphase am Kieferknochen - beispielsweise mittels Stiften, Nägeln, Schrauben oder auf andere Weise - fixiert werden.

Die exemplarisch genannten Fixiermittel können resorbierbar oder nicht resorbierbar sein.

Optional wird die Abdeckmembran nicht am Kieferknochen fixiert.

Die Abdeckmembran kann beispielsweise auf das Stützelement des erfindungsgemäßen zahntechnischen Implantats stirnseitig zur Mundhöhle hin (im implantierten Zustand) aufgelegt werden. Ein Fixieren oder Anhaften der Abdeckmembran an bzw. auf dem Stützelement kann beispielsweise mittels Bluts aus dem Operationsumfeld des Kieferknochens erfolgen.

Die Abdeckmembran kann eine flexible Folie sein. Die Abdeckmembran kann eine Membran, Abdeckfolie, Folie oder ein anderes Deckelement sein.

Die Abdeckmembran kann aus einem resorbierbaren oder einem nicht resorbierbaren Material hergestellt sein.

Ein resorbierbares Material kann, wie hierin verwendet, als selbstauflösendes Material bezeichnet werden, da es biologisch abgebaut und somit quasi aufgelöst wird. Ein resorbierbares Material kann rein exemplarisch Kollagen sein oder Kollagen umfassen.

Ein nicht resorbierbares Material, wie hierin genannt, kann ein Kunststoff, insbesondere ein biokompatibler Kunststoff sein, beispielsweise Polytetrafluorethylen (PTFE).

Nach der Knochenaufbauphase, die einige Wochen oder Monate dauern kann, ist die das Deckelement oder die Abdeckmembran entweder resorbiert oder wird operativ wieder entfernt. Anschließend kann das Zahnimplantat, bei dem es sich nicht um das zahntechnische Implantat handelt, in den Kieferknochen und in den aufgebauten Knochen eingesetzt werden. Ziel des Knochenaufbaus mit der Abdeckmembran ist in der Regel eine erhöhte Stabilität und damit eine verbesserte Verankerung des Zahnimplantats in dem mittels des Knochenaufbaus vergrößerten oder augmentierten Kieferknochen.

Das zahntechnische Implantat kann in einigen erfindungsgemäßen Ausführungsformen in den Kieferknochen eingesetzt werden, und ist hierfür vorgesehen, bevor das Zahnimplantat an derselben Implantationsstelle, der Implantationsregion oder der Augmentationsstelle in den Kieferknochen eingesetzt wird.

Zum möglichst ungestörten, knöchernen Durchwachsen des Knochenaufbaumaterials zwischen dem Kieferknochen und der Abdeckmembran kann das erfindungsgemäße zahntechnische Implantat mittels einer Stützstruktur einen Hohlraum ausbilden bzw. erzeugen. Dieser Hohlraum wird insbesondere geometrisch begrenzt durch den Kieferknochen mit dem aufliegenden Knochenaufbaumaterial und der Stützstruktur mit der Abdeckmembran.

Beispielsweise kann das erfindungsgemäße zahntechnische Implantat schirmförmig aufgebaut sein, wobei der Schirmmast dem Verbindungselement, die Streben im Schirm zur Befestigung der Schirmbespannung dem Stützelement und die Schirmbespannung der Abdeckmembran entsprechen.

In manchen Ausführungsformen ist das erfindungsgemäße zahntechnische Implantat ein temporäres Implantat für den Kieferknochen. Das temporäre zahntechnische Implantat kann beispielsweise einige Wochen oder einige Monate implantiert sein oder bleiben. Wenn das zahntechnische Implantat aus einem resorbierbaren Material hergestellt wurde, ist in der Regel keine weitere Operation zu seinem Entfernen notwendig, sondern das Implantat wird selbstständig resorbiert. Wenn das zahntechnische Implantat aus einem nicht resorbierbaren Material hergestellt wurde, wird es in einer weiteren Operation aus dem Kieferknochen wieder entfernt bzw. explantiert. Anschließend kann das Zahnimplantat in der oder in die Implantationsstelle oder -region, usw. implantiert werden, in die zunächst das zahntechnische Implantat implantiert worden war.

Im Gegensatz zu dem in einigen Ausführungsformen temporären zahntechnischen Implantat ist ein Zahnimplantat, wie es optional zusätzlich zum zahntechnischen Implantat Teil des erfindungsgemäßen Sets sein kann, dafür ausgestaltet, um möglichst dauerhaft in dem Kieferknochen zu verbleiben. Ein dauerhaftes Zahnimplantat kann beispielsweise mindestens zehn Jahre in dem Kieferknochen verbleiben. In einigen Fällen verbleibt das Zahnimplantat zwanzig Jahre, dreißig Jahre oder länger im Kieferknochen. Es kann eine Zahnkrone tragen oder hierzu vorbereitet sein.

Das Verbindungselement kann eine längliche Form mit einem innen hohlen oder nicht hohlen Querschnitt aufweisen. Der Querschnitt senkrecht zur Längsausrichtung kann rund, oval, eckig oder anders ausgestaltet sein. Der zweite Endabschnitt des Verbindungselements zum Verbinden des Implantats mit der Implantationsstelle des Kieferknochens kann spitz zulaufen, ähnlich einer Nagelform. Diese Form des zweiten Endabschnitts kann ein einfaches Befestigen des Verbindungselements ermöglichen, da es mittels Eindrücken oder Einschlagen in den Kieferknochen eingebracht werden kann.

Das Verbindungselement oder dessen zweiter Endabschnitt kann ein Gewinde, insbesondere ein Außengewinde, zum Einschrauben bzw. Eindrehen des Verbindungselements, des zweiten Endabschnitts oder zumindest eines Bereichs des zweiten Endabschnitts, in eine Implantationsstelle des Kieferknochens aufweisen. In anderen Ausführungsformen weist das Verbindungselement und/oder dessen zweiter Endabschnitt ein Gewinde nicht auf und/oder ist nicht zum Einschrauben in den Kieferknochen vorgesehen.

Das Verbindungselement kann in dessen Längsrichtung sich erstreckende Schlitze aufweisen.

Der Außendurchmesser eines runden Verbindungselements kann rein exemplarisch zwischen 0,5 mm und 2 mm betragen, beispielsweise 0,8 mm, 0,9 mm oder 1 mm. Die Gesamtlänge des Verbindungselements kann rein exemplarisch zwischen 5 mm und 20 mm betragen, beispielsweise eine Schaftlänge von 10 mm, eine Länge des ersten Endabschnitts von ca. 0,3 mm und eine Länge des zweiten Endabschnitts von ca. 3 mm.

Das erste Stützelement kann als Gitterelement und/oder als Versteifungselement ausgeführt sein.

In einigen Ausführungsformen weist das erste Stützelement eine gitterförmige Struktur oder einen gitterförmigen Abschnitt auf.

Eine gitterförmige Struktur oder ein gitterförmiger Abschnitt kann beispielsweise eine rechteckige Außenkontur mit innen angeordneten Längsstreben und Querstreben aufweisen. Beispielsweise kann das Gitterelement eine mittige Längsstrebe und drei, fünf oder mehr Querstreben aufweisen.

Diese oder anders gestaltete oder angeordnete innere Streben können rechtwinklig oder in einem anderen Winkel zueinander angeordnet sein.

Die inneren Streben können an einem äußeren, geschlossenen Rand befestigt sein. Rein exemplarisch kann die rechteckige Außenkontur eine Länge von ca. 10 mm und eine Breite von ca. 5 mm aufweisen.

Die rechteckige Außenkontur kann abgerundete Ecken aufweisen.

Die gitterförmige Struktur kann alternativ eine (z. B. in Draufsicht) runde, eine ovale oder eine andere Form als Außenkontur aufweisen. Eine runde Form kann rein exemplarisch einen Außendurchmesser von ca. 5 mm aufweisen.

Eine runde Form des Gitterelements kann sternförmig angeordnete bzw. radiale Streben aufweisen, beispielsweise drei, vier, fünf oder mehr Streben. Die sternförmigen Streben können untereinander regelmäßig oder unregelmäßig hinsichtlich des Winkelabstands zwischen sich angeordnet sein.

In manchen Ausführungsformen ist der zweite Verbindungsabschnitt zentral mittig innerhalb des ersten Stützelements angeordnet oder mit diesem verbunden.

Das erste Stützelement kann als Spritzgussteil hergestellt sein.

In einigen Ausführungsformen entsprechen der erste Verbindungsabschnitt des Verbindungselements und der zweite Verbindungsabschnitt des Stützelements einander (z. B. als Teile eines Klicksystems, eines Klemmsystems, als Nut und Feder, als Schraube und Mutter, usw.) und/oder sind zur gemeinsamen Verbindung miteinander aufeinander abgestimmt.

Der erste oder der zweite Verbindungsabschnitt ist vorzugsweise als Einstecköffnung ausgestaltet, wobei der andere dieser beiden Verbindungsabschnitte als Einsteckstift ausgestaltet ist.

Beispielsweise ist der erste Verbindungsabschnitt des Verbindungselements, der dem ersten Endabschnitt des Verbindungselements entspricht, als Einsteckstift ausgestaltet. In diesem Fall ist der zweite Verbindungsabschnitt des Stützelements als Einstecköffnung ausgestaltet.

Die Verbindung zwischen dem Einsteckstift und der Einstecköffnung kann beispielsweise als Übergangspassung oder als Presspassung ausgeführt sein, um ein selbstständiges und ungewolltes Lösen des Verbindungselements von dem Stützelement zu verhindern.

Der Einsteckstift und die Einstecköffnung können über der Länge einen gleichen Durchmesser aufweisen. Alternativ kann sich der Einsteckstift zur Spitze hin verjüngen und die Einstecköffnung eine entsprechend konische Form aufweisen. Dies kann ein Ineinanderstecken der beiden Teile erleichtern.

In manchen Ausführungsformen ist der Implantationsabschnitt als zulaufender oder sich verjüngender Abschnitt ausgestaltet. Dies kann ein Befestigen des Verbindungselements im Kieferknochen durch ein Einschlagen oder ein Eindrücken erleichtern.

In einigen Ausführungsformen sind der erste Verbindungsabschnitt und der zweite Verbindungsabschnitt zur lösbaren Verbindung miteinander ausgestaltet. Dies ermöglicht einen modularen Aufbau des Verbindungselements mit dem Stützelement, sodass unterschiedliche Ausführungsformen miteinander kombiniert werden können. Beispielsweise kann es je nach Form und Größe des Knochenaufbaus auf dem Kieferknochen erforderlich sein, ein langes oder kurzes, ein dickes oder dünnes Verbindungselement auszuwählen. Je nach der gewünschten oder möglichen Oberfläche des Knochenaufbaus kann es vorteilhaft sein, entsprechend große Stützelemente auszuwählen und mit einem ausgewählten Verbindungselement zu kombinieren.

In einigen Ausführungsformen weist das erste Stützelement eine im bestimmungsgemäßen Implantationszustand des Implantats der Mundhöhle des Patienten zugewandte Oberseite und eine der Implantationsstelle zugewandte Unterseite auf. Der zweite Verbindungsabschnitt ist vorzugsweise derart ausgestaltet, um den ersten Verbindungsabschnitt von seiner Unterseite her aufzunehmen. Auf der Oberseite des ersten Stützelements kann die Abdeckmembran aufliegen. Ein mechanisches Befestigen der Abdeckmembran, beispielsweise am ersten Stützelement und/oder am Kieferknochen ist oftmals nicht notwendig. Stattdessen kann ein Verkleben mittels Blut aus dem Operationsumfeld des Kieferknochens für ein Anhaften der Abdeckmembran an oder auf dem Stützelement ausreichend sein.

Je nach individueller Situation kann zusätzlich ein Verankern der Abdeckmembran am Kieferknochen vorgenommen werden.

Ein Verankern kann beispielsweise mittels eines oder mehreren resorbierbaren oder nicht resorbierbaren Verankerungsstiften bzw. Nägeln im Kieferknochen erfolgen.

Ein resorbierbarer Verankerungsstift kann beispielsweise aus Magnesium hergestellt sein oder Magnesium aufweisen, ein nicht resorbierbarer Verankerungsstift kann beispielsweise aus Titan oder einer Titanlegierung gefertigt sein oder ein solches Material aufweisen.

In manchen Ausführungsformen weist das Verbindungselement keinen Gewindeabschnitt und/oder keinen Schraubenkopf auf.

In einigen Ausführungsformen ist das Verbindungselement und/oder das erste Stützelement aus einem Metall, einem Kunststoff und/oder einem Verbundmaterial hergestellt oder weist ein solches Material auf. Die genannten Materialien sind insbesondere biokompatibel.

In manchen Ausführungsformen ist das Verbindungselement und/ oder das erste Stützelement aus einem resorbierbaren Material hergestellt oder weist ein solches Material auf.

Das Verbindungselement kann z. B. aus einem resorbierbaren Magnesium oder magnesiumhaltigen Material hergestellt sein oder ein solches aufweisen, wie es beispielsweise in den Patentschriften EP 2 744 531 B1 und EP 2 744 532 B1 beschrieben ist.

Das Stützelement kann rein exemplarisch aus einem Polylactid hergestellt sein oder ein solches aufweisen.

In einigen Ausführungsformen weist der erste Verbindungsabschnitt einen konischen Bereich auf, auf welchen das Stützelement aufgesteckt werden kann. Je nach der verwendeten Materialpaarung des Verbindungselements einerseits und des Stützelements andererseits kann der Konuswinkel $\alpha$ derart gewählt werden, dass eine selbsthemmende Kegelpressverbindung zwischen Verbindungselement und Stützelement entsteht.

Das Werkzeug zum Einschlagen des Verbindungselements in den Kieferknochen kann einen umlaufenden Spaltabschnitt aufweisen, welcher der Aufnahme eines rohr- oder ringförmigen Endbereichs des Stützelements, insbesondere dessen ersten Verbindungsabschnitts oder eines Abschnitts oder Endabschnitts hiervon dienen kann.

Der Spaltabschnitt kann an seiner äußeren Begrenzung und/oder seiner inneren Begrenzung von zylindrischen Strukturen unterschiedlicher Höhe begrenzt sein.

Die Höhe des Spalts kann größer als die Länge eines Konus des Verbindungselements sein.

Das Werkzeug kann als Werkzeug für den Zahnarzt oder Kieferchirurgen eine Länge von weniger als 3 cm, weniger als 5 cm oder weniger als 10 cm haben. Es kann unabhängig von seiner Länge eine Breite oder einen Durchmesser von weniger als 2 cm, weniger als 1 cm oder weniger als 0,5 cm aufweisen.

In einigen Ausführungsformen weist das Stützelement einen zentralen Bereich mit einer Öffnung zum Einstecken oder Hindurchführen eines endseitigen Abschnitts des Verbindungselements auf.

Das Stützelement kann eine Anzahl von z. B. radial nach außen verlaufenden oder ausgerichteten Streben oder Stege zwischen einem inneren, vorzugsweise scheibenförmigen, Bereich und z. B. einem äußeren Ring oder einer, allgemeiner formuliert, äußeren Struktur des Stützelements aufweisen.

Zur Befestigung des Verbindungselements am Stützelement sind erfindungsgemäß ein oder mehrere Stifte vorzugsweise am Verbindungselement vorgesehen, optional als einzige oder alleinige Verbindungsstrukturen. Sie erstrecken sich erfindungsgemäß endseitig vom Verbindungselement und sind verbiegbar.

Die Herstellung des Stützelements kann beispielsweise aus einem scheibenförmigen oder rohrförmigen Halbzeug als Ausgangsmaterial erfolgen, das mittels Laserschneiden bearbeitet wird.

Zur exakten Positionierung des Stützelements am Verbindungselement während des Montierschritts kann die innere Scheibe des Stützelements Einkerbungen zum Führen der Stifte aufweisen, sie können in axialer Richtung verlaufen, also senkrecht zur radialen Richtung, in welcher die Streben sich erstrecken.

Das Verbindungselement oder seine optionale innere Scheibe kann eine, vorzugsweise zentrale, Öffnung aufweisen. Diese kann dimensioniert sein, um mit ihrem Rand auf einem optional vorgesehenen Rand oder Absatz, aufgesetzt zu werden. Eine stirnseitige Ausgestaltung des Verbindungselements kann somit in oder durch die Öffnung hinein- oder hindurchragen. Die Öffnung kann als Sacköffnung oder als Durchgangsöffnung ausgestaltet sein.

Einer oder mehrere der oben stehenden sowie der folgenden Vorteile können mittels der vorliegenden Erfindung erzielt werden.

Das erfindungsgemäße zahntechnische Implantat kann vollständig oder teilweise aus resorbierbaren Materialien gefertigt sein. Dadurch kann vorteilhaft ein chirurgischer bzw. operativer Zweiteingriff vermieden werden, wie er bei der notwendigen Entfernung nicht resorbierbarer Metalle oder Kunststoffe notwendig wäre. Ein chirurgischer Eingriff am Kieferknochen ist in der Regel eine erhebliche Belastung und ein mögliches Risiko, beispielsweise in Form eines Infektionsrisikos, für den Patienten. Außerdem ist ein chirurgischer Eingriff i.d.R. mit erheblichen Kosten verbunden.

Wenn weiterhin die Abdeckmembran und/oder mögliche Befestigungsmittel der Abdeckmembran am Kieferknochen ebenfalls aus resorbierbaren Materialien hergestellt sind, kann dieser Vorteil noch weiter gesteigert werden.

Das, vorzugsweise modulare zweiteilige (oder optional wenigstens zweiteilige oder mehrteilige), erfindungsgemäße zahntechnische Implantat mit dem Verbindungselement als erstem Bauteil und dem Stützelement als zweitem Bauteil ermöglich vorteilhaft die Kombination unterschiedlicher Verbindungselemente und Stützelemente. Je nach patientenindividueller und anatomischer Situation kann es während eines operativen Eingriffs notwendig sein, von den gewählten Größen und Modellen des Verbindungselements und/oder des Stützelements abzuweichen. Dadurch kann vorteilhaft eine optimale Kombination ausgewählt werden, die für einen späteren Erfolg einer dauerhaften Implantation eines Zahnimplantats (als künstliche Zahnwurzel für den Aufbau und die Krone, also der prothetischen Versorgung) entscheidend sein kann. Somit können z. T. erhebliche Folgekomplikationen, verbunden mit einer hohen Belastung für den Patienten und erhebliche Kosten vorteilhaft vermieden oder zumindest verringert werden.

Das erfindungsgemäße zahntechnische Implantat ist eine vorteilhaft einfache und kostengünstige Alternative gegenüber patientenindividuellen Anfertigungen, wie sie beispielsweise aufgrund anatomischer Gegebenheiten mittels bildgebender Verfahren und anschließender generativer Herstellungsmethoden derzeit möglich sind und angewendet werden.

Das erfindungsgemäße Verbindungselement ermöglicht ein vorteilhaft einfaches Befestigen des zahntechnischen Implantats im Kieferknochen. Eine aufwendige Verschraubung im Kieferknochen kann dadurch vermieden werden.

Die vorliegende Erfindung wird im Folgenden anhand der beigefügten, zum Teil vereinfachten Figuren, in welchen identische Bezugszeichen gleiche oder ähnliche Bauteile bezeichnen, exemplarisch erläutert. In den Figuren gilt:

**Fig. 1** zeigt einen Knochenaufbau am Kieferknochen zur Vorbereitung einer Implantation von zwei Zahnimplantaten;

**Fig. 2a** zeigt ein erstes Stützelement eines erfindungsgemäßen zahntechnischen Implantats;

**Fig. 2b** zeigt ein zweites Stützelement eines erfindungsgemäßen zahntechnischen Implantats;

**Fig. 2c** zeigt ein drittes Stützelement eines erfindungsgemäßen zahntechnischen Implantats;

**Fig. 2d** zeigt ein Stützelement eines erfindungsgemäßen zahntechnischen Implantats in einer Seitenansicht;

**Fig. 3a** zeigt ein Verbindungselement eines erfindungsgemäßen zahntechnischen Implantats;

**Fig. 3b** zeigt ein weiteres Verbindungselement eines erfindungsgemäßen zahntechnischen Implantats;

**Fig. 3c-k** zeigen weitere Ausführungsformen des Verbindungselements;

**Fig. 4** zeigt ein erfindungsgemäßes zahntechnisches Implantat mit Stützelement und Verbindungselement;

**Fig. 4a** zeigt eine weitere Ausführungsform eines erfindungsgemäßes zahntechnisches Implantats;

**Fig. 5** zeigt den Knochenaufbau am Kieferknochen aus Fig. 1 mit einem erfindungsgemäßen zahntechnischen Implantat;

**Fig. 6a-c** zeigen ein Werkzeug zum Einschlagen des Verbindungselements in den Kieferknochen; und

**Fig. 7** zeigt eine Fertigungsvariante für das Stützelement.

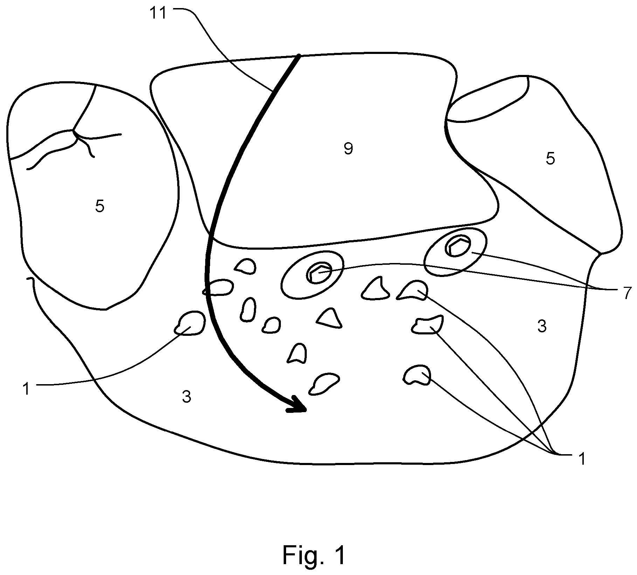

**Fig. 1** zeigt einen Knochenaufbau mittels Knochenersatzmaterial 1 am Kieferknochen 3 zur Vorbereitung einer Implantation von zwei Zahnimplantaten. Das Knochenersatzmaterial 1 ist exemplarisch als Granulat ausgeführt. Das optional granulierte Knochenersatzmaterial 1 kann synthetisches Knochenersatzmaterial 1, beispielsweise in Form von Hydroxylapatit, sein und/oder natürliches, körpereigenes Knochenersatzmaterial 1. Oft werden diese beiden Formen, also synthetisches und körpereigenes Material, miteinander gemischt.

Das Knochenersatzmaterial 1 ist exemplarisch auf dem Kieferknochen 3 aufgelegt und verteilt angeordnet. Die Anordnung ist rein exemplarisch und grob schematisch dargestellt. Tatsächlich ist das Granulat in der Praxis oft wesentlich dichter und auch übereinander geschichtet angeordnet. Ziel dieser geschichteten Anordnung von Knochenersatzmaterial 1 ist ein knöchernes Durchwachsen, ausgehend von dem Kieferknochen 3. Nach Abschluss dieses Knochenwachstums, das in der Regel mehrere Monate dauert, kann der Kieferknochen zusammen mit dem neu gebildeten Knochen die knöcherne Basis zum Implantieren eines Zahnimplantats bilden. Das Zahnimplantat, das als künstliche Zahnwurzel bezeichnet werden kann, ist anschließend die Basis für den weiteren Aufbau mittels eines sogenannten *abutment* und einer sich daran anschließenden Zahnkrone. Aufgrund dieser Beschreibung wird deutlich, dass ein stabiles und ausreichendes Knochenlager für das Zahnimplantat die Basis für einen dauerhaften Erfolg, das heißt ein möglichst langes Verbleiben des Zahnimplantats im Knochen, bildet.

In Fig. 1 sind weiterhin zwei intakte Zähne 5 dargestellt, zwischen denen optional zwei Schrauben 7 als Platzhalter für das spätere Zahnimplantat in den Kieferknochen 3 eingeschraubt sind. Weiterhin ist eine exemplarische Abdeckmembran 9 dargestellt, die in Pfeilrichtung 11 auf das Granulat 1 aufgelegt wird. Die Abdeckmembran 9, die als Abdeckfolie oder als Deckelement bezeichnet bzw. ausgeführt werden kann, hat die Funktion, das Knochenersatzmaterial 1 während des knöchernen Durchwachsens gegen ein unerwünschtes Einwachsen von Weichgewebe, beispielsweise von umgebender Schleimhaut, zu schützen. Ein derartiges Einwachsen von Weichgewebe würde die knöcherne Struktur in ihrer Stabilität und Festigkeit deutlich einschränken und soll daher mittels der Abdeckmembran 9 verhindert werden. Allerdings ist ein direktes Aufliegen der Abdeckmembran 9 auf dem Granulat 1 für das knöcherne Durchwachsen nachteilig, da sich der Druck der Abdeckmembran 9 störend und nachteilig auf das Durchwachsen auswirkt.

Die Abdeckmembran 9 kann beispielsweise mittels Befestigungsstiften am Rand der Abdeckmembran 9 an dem Kieferknochen 3 fixiert werden. Als Befestigungsstifte können Nägel, Schrauben oder Ähnliches verwendet werden, die resorbierbar oder nicht resorbierbar sein können.

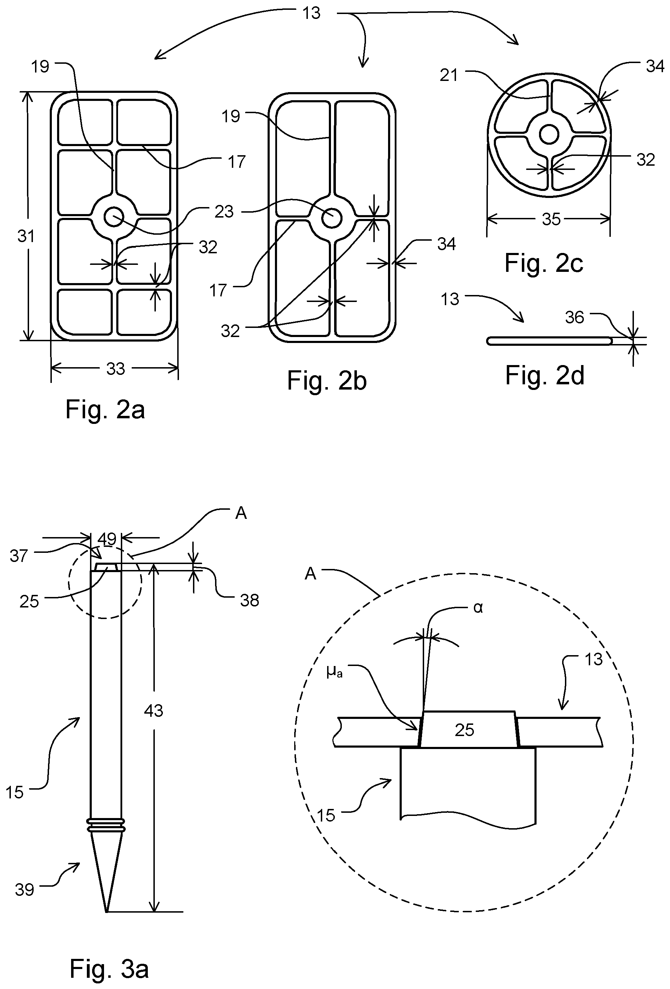

In den **Fig. 2a, 2b, 2c** werden exemplarisch unterschiedlich ausgestaltete Stützelemente 13 eines erfindungsgemäßen zahntechnischen Implantats 100 dargestellt.

Die Stützelemente 13 dienen dem Abstützen der Abdeckmembran 9, um zusammen mit dem Verbindungselement 15 einen Hohlraum zwischen der Abdeckmembran 9 und dem Knochenersatzmaterial 1 auszubilden. In diesem Hohlraum kann das knöcherne Durchwachsen ungestört stattfinden. Die Abdeckmembran 9 wird auf der Oberseite des Stützelements 13 aufgelegt, wobei die Oberseite die zur Mundhöhle hin ausgerichtete Seite des Stützelements 13 ist. Demgegenüber ist die Unterseite des Stützelements 13 zum Kieferknochen 3 hin ausgerichtet. Nach dem Auflegen wird die Abdeckmembran 9 optional mittels Blut bzw. dem darin befindlichen Fibrin an dem Stützelement 13 befestigt bzw. angeklebt. Ein weiteres optionales Fixieren der Abdeckmembran 13 am Kieferknochen 3 ist meistens nicht notwendig.

Die unterschiedlichen geometrischen Formen der verschiedenen Stützelemente 13 in Fig. 2a, Fig. 2b und Fig. 2c können je nach Größe der Abdeckmembran 9 bzw. nach Größe des aufzubauenden Knochens, der jeweiligen anatomischen Situation und/oder anderen Einflussfaktoren ausgewählt werden. Dabei ist es besonders vorteilhaft, dass die modulare Bauweise des erfindungsgemäßen zahntechnischen Implantats es erlaubt, die beiden Modulelemente Verbindungselement 15 einerseits und Stützelement 13 andererseits beliebig hinsichtlich Größe usw. miteinander zu kombinieren. Dies bedeutet, dass noch während einer chirurgischen Operationssituation das am besten geeignete Exemplar eines Verbindungselements 15 zusammen mit dem am besten geeigneten Exemplar des Stützelements 13 ausgewählt und verbunden werden kann. Dies ermöglicht eine bestmögliche Versorgung.

Der Unterschied zwischen den beiden Stützelementen 13 in den Fig. 2a und 2b besteht in der unterschiedlichen Anzahl von Querstreben 17. Eine höhere Anzahl von Querstreben 17 kann in einer erhöhten Steifigkeit gegen ein Verbiegen resultieren. Die Steifigkeit bzw. Biegesteifigkeit hängt allerdings noch von weiteren Faktoren ab, wie beispielsweise der Querschnittsform der Querstreben 17 und den jeweiligen Materialeigenschaften. Je nach der geometrischen Ausgestaltung der Stützelemente 13 kann somit die Größe des Hohlraums zwischen der Abdeckmembran 9 und dem Kieferknochen 3 beeinflusst werden. Dies hat wiederum einen direkten Einfluss auf den sich ausbildenden Kieferknochen und somit auf die Stabilität des späteren Zahnimplantats. Für die Längsstreben 19 und die radialen Streben 21 in der runden Ausführungsform in Fig. 2c gelten die Aussagen in analoger Form.

Optional zentral mittig in den Stützelementen 13 sind je eine oder mehrere Einstecköffnungen 23 angeordnet, in denen ein Einsteckstift 25 des Verbindungselements 15 eingeführt werden kann. Der Einsteckstift 25 kann als erster Verbindungsabschnitt 25 des Verbindungselements 15 bezeichnet werden, die Einstecköffnung 23 als zweiter Verbindungsabschnitt 23 des Stützelements 13.

Die Länge 31 des Stützelements 13 kann rein exemplarisch ca. 10 mm betragen, die Breite 33 rein exemplarisch ca. 5 mm. Weiterhin kann rein exemplarisch die Dicke 32 der Querstreben 17 und/oder der Längsstreben 19 ca. 0,2 mm betragen, die Dicke 34 der äußeren, umlaufenden Streben ca. 0,25 mm. Der Durchmesser der Einstecköffnung 23 kann rein exemplarisch ca. 0,8 mm betragen. Der äußere Durchmesser 35 der runden Ausführungsform des in Fig. 2c dargestellten Stützelements kann rein exemplarisch ca. 5 mm betragen.

**Fig. 2d** zeigt ein Stützelement 13 eines erfindungsgemäßen zahntechnischen Implantats 100 in einer Seitenansicht, wobei das Stützelement 13 beispielsweise eine der dargestellten Ausführungsformen in Fig. 2a, Fig. 2b oder Fig. 2c sein kann. Die - optional zumindest abschnittsweise oder über wenigstens die halbe Breite gleichbleibende - Höhe 36 des Stützelements 13 kann rein exemplarisch einen Wert zwischen 0,1 mm und 0,2 mm aufweisen, beispielsweise 0,16 mm.

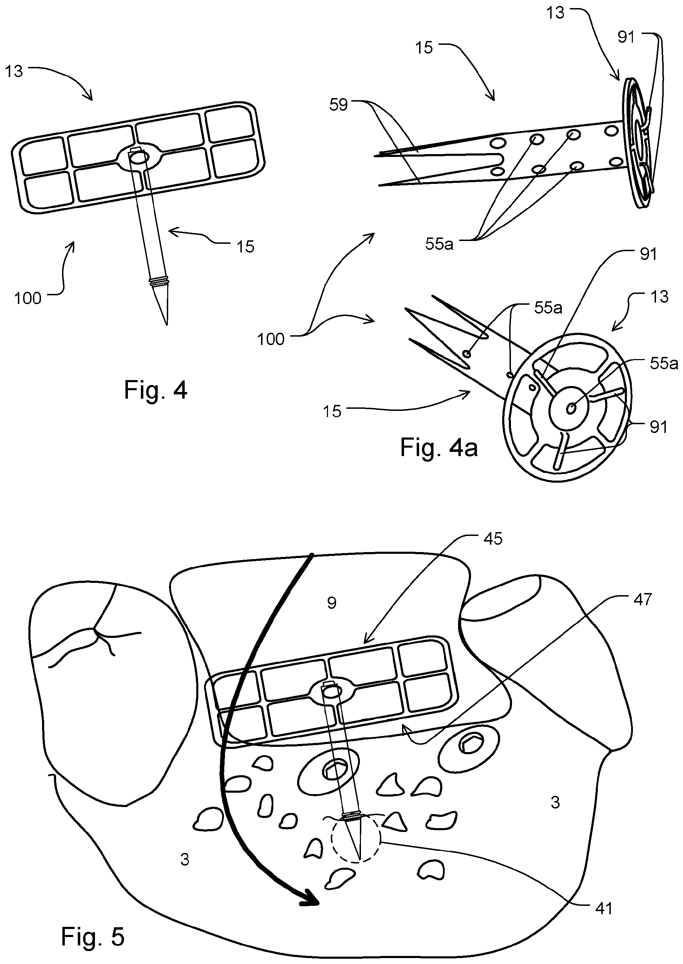

**Fig. 3a** zeigt ein Verbindungselement 15 eines erfindungsgemäßen zahntechnischen Implantats 100 mit einem ersten Endabschnitt 37 und einem zweiten Endabschnitt 39 zum Verbinden des Implantats 100 mit einer Implantationsstelle 41 (siehe Fig. 5) eines Kieferknochens 3.

Die Gesamtlänge 43 des Verbindungselements 15 kann rein exemplarisch ca. 13 mm betragen, wobei der erste Endabschnitt 37, der als Einsteckstift 25 bzw. als erster Verbindungsabschnitt 25 ausgestaltet ist, rein exemplarisch eine Länge 38 von ca. 0,3 mm und der zweite Endabschnitt 39 rein exemplarisch eine Länge von ca. 3 mm aufweist. Der Außendurchmesser 49 eines optional runden Verbindungselements 15 kann rein exemplarisch ca. 1,2 mm betragen.

In der vergrößerten Darstellung des Ausschnitts A der Fig. 3a, in der zusätzlich ein Abschnitt des aufgesteckten Stützelements 13 dargestellt ist, ist ein Konuswinkel $\alpha$ des sich längsseitig oder in Längsrichtung verjüngenden ersten Verbindungsabschnitts 25 dargestellt.

Dieser Konuswinkel $\alpha$ kann analog in der Einstecköffnung 23 des Stützelements 13 gefertigt bzw. ausgeführt werden. Somit kann vorteilhaft einfach das Stützelement 13 auf den ersten Verbindungsabschnitt 25 aufgeschoben werden.

Je nach der optional gewählten Materialpaarung des ersten Verbindungsabschnitts 25 und des Stützelements 13 resultiert daraus ein Reibungskoeffizient $\mu$.

Der Reibungskoeffizient $\mu$ kann in unterschiedlichen Richtungen entlang der Gleitfläche unterschiedliche Werte des Reibungskoeffizienten $\mu$ aufweisen, beispielsweise abhängig von einer strukturierten Oberfläche. Exemplarisch für diese unterschiedlichen Richtungen wird nachfolgend eine Axialrichtung und eine senkrecht zur Axialrichtung ausgerichtete Tangentialrichtung angegeben. In der Axialrichtung, die insbesondere der Bewegungsrichtung des Stützelements 13 relativ zum Verbindungsabschnitt 25 entspricht, kann ein Reibungskoeffizient in Axialrichtung $\mu_a$ angegeben werden.

Der Reibungskoeffizient $\mu$ kann als Reibwert oder Reibungszahl bezeichnet werden und ist ein dimensionsloses Maß für die Reibungskraft im Verhältnis zur Anpresskraft zwischen zwei Körpern. Die Reibungskraft wirkt parallel zur Kontaktfläche und hängt von dem material- und oberflächenabhängigen Reibungskoeffizienten $\mu$ und einer senkrecht zur Kontaktfläche wirkenden Normalkraft ab.

Nach dem bekannten Zusammenhang für Kegelverbindungen bei Maschinenelementen kann daraus ein notweniger Konuswinkel $\alpha$ für eine selbsthemmende Kegelpressverbindung berechnet werden. Danach gilt die folgende Ungleichung für eine selbsthemmende Verbindung zwischen dem ersten Verbindungsabschnitt 25 des Verbindungselements 15 und dem Stützelement 13.

Selbsthemmung: $\alpha < \arctan \mu_a$

$\alpha$:     Konuswinkel

$\mu_a$:     Reibungskoeffizient in Axialrichtung

arctan:     Arkustangens, Umkehrfunktion der trigonometrischen Tangensfunktion

Der Reibungskoeffizient $\mu$ kann in Axialrichtung und in Tangentialrichtung, die beide parallel zur Gleitfläche verlaufen, unterschiedlich sein. Für die Selbsthemmung in Axialrichtung wird daher für die oben genannte Ungleichung nur der Reibungskoeffizient in Axialrichtung betrachtet.

Der Reibungskoeffizient $\mu$ wird durch die Materialpaarung bestimmt und kann unterschiedlich groß sein. Beispielsweise beträgt der Haftreibungskoeffizient $\mu$ für eine trockene, also ungeschmierte, Materialpaarung Stahl auf Stahl zwischen ca. 0,15 und 0,3. Für einen Wert von $\mu$ = 0,15 kann somit der notwendige Konuswinkel für eine selbsthemmende Verbindung wie folgt berechnet werden:

$$\alpha < \arctan 0,15 \qquad\qquad\qquad (1)$$
$$\alpha < 8,5° \text{ (Grad)}$$

Für einen rein exemplarisch gewählten Konuswinkel von $\alpha$ = 5,71° wäre dies somit für eine Materialpaarung Stahl auf Stahl eine selbsthemmende Kegelpressverbindung. Damit kann vorteilhaft eine sichere Fixierung bzw. Verbindung zwischen dem ersten Verbindungsabschnitt 25 des Verbindungselements 15 und dem Stützelement 13 hergestellt werden.

Ein solcher Konuswinkel $\alpha$ von weniger als ca. 11 Grad oder ein nach vorstehender Formel (1) (mit konkretem Wert $\mu$ statt wie dort exemplarisch verwendet 0,15) berechneter Konuswinkel $\alpha$ ist in einigen Ausführungsformen vorgesehen.

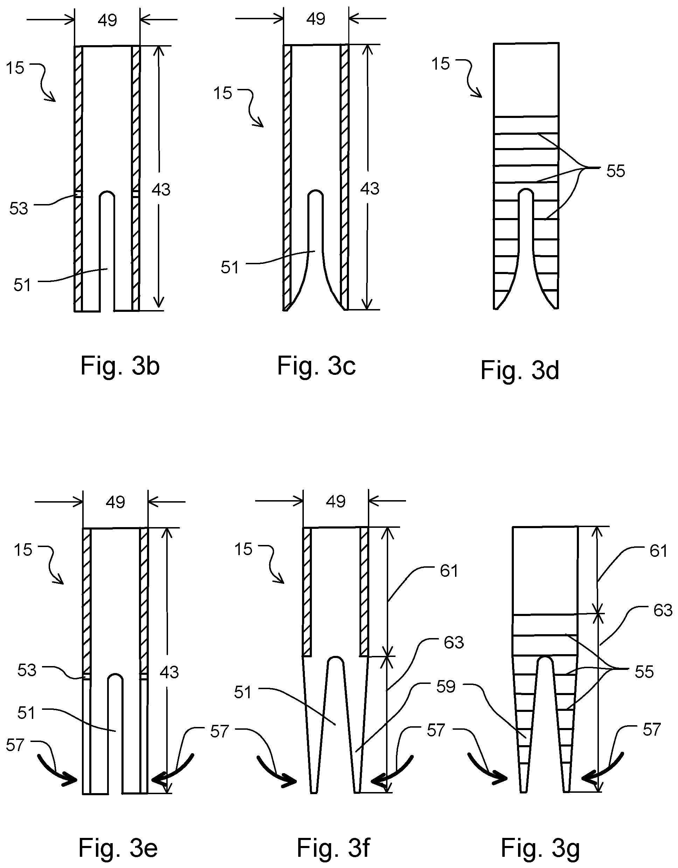

**Fig. 3b** zeigt ein weiteres Verbindungselement 15 eines erfindungsgemäßen zahntechnischen Implantats 100 in einer Längsschnittdarstellung. Im Gegensatz zu der Ausführungsform in Fig. 3a ist das weitere Verbindungselement 15 in Fig. 3b rohrförmig, innen hohl und mit einem optional größeren oder kleineren Außendurchmesser 49 gegenüber dem Verbindungselement 15 aus Fig. 3a ausgeführt. Dadurch kann eine höhere Festigkeit bzw. Biegesteifigkeit gegen ein unbeabsichtigtes Verbiegen oder Abknicken, insbesondere bei einem Einschlagen des Verbindungselements 15 in den Kieferknochen 3 ermöglicht werden.

Das weitere Verbindungselement 15 weist in der unteren Hälfte einen, zwei oder mehr Schlitze 51 auf, die, falls zu zweien vorgesehen, in Umfangsrichtung um 180 Grad versetzt angeordnet sind. Diese Schlitze 51 ermöglichen es, die untere Hälfte mittels eines Zusammendrückens des rohrförmigen Verbindungselements 15 bzw. einem Zusammenquetschen das untere Ende annähernd spitzförmig zu formen, sodass ein Einbringen bzw. ein Einschlagen in den Kieferknochen 3 vereinfacht wird.

Dieses Zusammendrücken kann durch - z. B. zwei - Öffnungen oder Bohrungen 53, die in Umfangsrichtung um 180 Grad versetzt angeordnet sind, vereinfacht werden. Die beiden Bohrungen 53 ermöglichen ein Abknicken des Verbindungselements 15 bei einem Zusammendrücken genau an dieser Stelle. Im Bereich dieser Bohrungen kann in dieser Ausführungsform eine plastische Verformung des Materials stattfinden bzw. mittels Finite-Elemente-Analyse nachgewiesen werden. Das zusammengedrückte untere Ende des weiteren Verbindungselements 15 kann somit in etwa dem zweiten Endabschnitt 39 des Verbindungselements 15 aus Fig. 3a entsprechen.

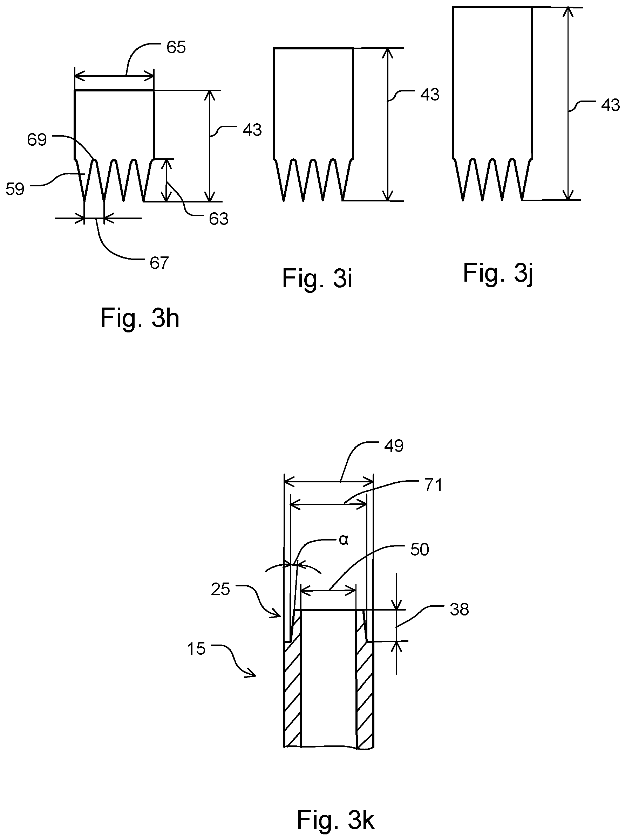

Zum Verbinden des weiteren Verbindungselements 15 mit einem Stützelement 13 kann beispielsweise ein Absatz in dem Stützelement 13 in das obere Ende des rohrförmigen weiteren Verbindungselements 15 eingeführt werden. Alternativ kann sich ein konusförmiger Abschnitt als erstem Verbindungsabschnitt an dem stirnseitigen Ende anschließen, der analog dem ersten Verbindungsabschnitt 25 des Verbindungselements 15 (siehe Fig. 3a) ausgeführt sein kann. Dies wird zu Fig. 3h näher beschrieben.

Die Gesamtlänge 43 des weiteren Verbindungselements 15 kann rein exemplarisch zwischen 7 mm und 15 mm , beispielsweise ca. 10 mm, betragen.

Der Außendurchmesser 49 des weiteren Verbindungselements 15 kann rein exemplarisch zwischen ca. 1 mm und 2 mm betragen, beispielsweise 1,8 mm.

Die Wandstärke des weiteren rohrförmigen Verbindungselements 15 kann rein exemplarisch einen Wert zwischen ca. 0,1 mm und 0,2 mm aufweisen, beispielsweise 0,16 mm.

**Fig. 3c** zeigt eine weitere Ausführungsform des Verbindungselements 15 im Längsschnitt mit spitz zulaufenden bzw. sich verjüngenden Abschnitten längs der beiden Schlitze 51. Die z. B. um 180 Grad im Umfangsrichtung versetzt angeordneten beiden Schlitze 51 (es könnten mehr oder weniger als zwei sein) verbreitern sich somit zum unteren, stirnseitigen Ende des Verbindungselements 15 hin. Damit können vorteilhaft die beiden stirnseitigen Endbereiche zusammengedrückt werden, um das Verbindungselement 15 einfach in den Kieferknochen einschlagen zu können.

Die Außenwände des Verbindungselements 15 können vollständig oder im Wesentlichen gerade verlaufen wie in Fig. 3b und 3c gezeigt. Die Innenwände können ganz oder zumindest teilweise gerade (wie in Fig. 3b gezeigt) und/oder teilweise gekrümmt (wie in Fig. 3c gezeigt) verlaufen.

**Fig. 3d** zeigt die Ausführungsform aus Fig. 3c in einer Seitenansicht ohne Schnittdarstellung. Dargestellt werden einzelne optionale Höhenmarkierungen 55 auf unterschiedlichen Höhen des Verbindungselements 15, die (alle oder manche) optional um den gesamten Umfang angeordnet werden können. Diese Höhenmarkierungen 55 können vorteilhaft dafür genutzt werden, um die Eindringtiefe in den Kieferknochen zu bestimmen. Die Höhenmarkierungen 55 können äquidistant oder nicht äquidistant angeordnet sein. Die Höhenmarkierungen 55 können alternativ oder ergänzend als Widerhaken ausgebildet sein, um ein festes Verankern des Verbindungselements 15 im Kieferknochen zu gewährleisten. Insbesondere bei einem optionalen, resorbierbaren Material des Verbindungselements 15 ist eine Explantation nicht notwendig und vorgesehen. Solche Höhenmarkierungen 55 können optional Teil jeder Ausführungsform sein.

**Fig. 3e** zeigt eine weitere Ausführungsform des Verbindungselements 15. Im Gegensatz zu den Ausführungsformen der Fig. 3b-d weisen die Verbindungselemente 15 der Fig. 3e-g nicht zwei, sondern vier über den Umfang angeordnete Abschnitte im unteren Bereich auf. Dies wird durch die nicht in Schnittdarstellung gezeigten unteren Bereiche verdeutlicht. Die vier Abschnitte können in Pfeilrichtung 57 zur Mitte hin gebogen werden, um ein Einschlagen in den Kieferknochen zu vereinfachen.

**Fig. 3f** zeigt eine weitere Ausführungsform des Verbindungselements 15, die ähnlich der Form aus Fig. 3e ausgebildet ist, jedoch keine Öffnungen oder Bohrungen 53 aufweist und spitzer zulaufende, untere Abschnitte aufweist. Die unteren Abschnitte können als Spitzen 59 bezeichnet werden. Ein Zusammendrücken bzw. plastisches Verformen der Spitzen 59 zur Mitte hin ermöglicht vorteilhaft eine gegenüber der Form aus Fig. 3e noch spitzer zulaufende Form, so dass ein Einschlagen in den Kieferknochen vereinfacht möglich ist.

Die obere Teillänge 61 und die untere Teillänge 63 des Verbindungselements 15 sind rein exemplarisch ungefähr jeweils halb so lang wie die Gesamtlänge 43.

**Fig. 3g** zeigt analog zur Fig. 3d eine Seitenansicht des Verbindungselements 15. Bezüglich der optionalen Höhenmarkierungen 55 wird daher auf die Beschreibung zur Fig. 3d verwiesen.

Die obere Teillänge 61, die in Fig. 3g den nicht-markierten Abschnitt des Verbindungselements 15 darstellt, entspricht rein exemplarisch ungefähr einem Drittel der Gesamtlänge 43. Die Oberfläche dieses oberen Abschnitts kann eine andere Oberflächenstruktur als der untere Abschnitt aufweisen, um beispielsweise ein verbessertes manuelles Festhalten zum Einschlagen des Verbindungselements 15 in den Kieferknochen zu ermöglichen.

In **Fig. 3h, Fig. 3i** und **Fig. 3j** sind schematisch vereinfacht Abwicklungen der in den Fig. 3f und Fig. 3g gezeigten Verbindungselemente 15 dargestellt. Die Länge der Abwicklung 65 entspricht dem Umfang. Bei einem rein exemplarischen äußeren Durchmesser von 1,8 mm errechnet sich die Länge der Abwicklung zu ca. 5,7 mm. Die untere Teillänge 63 des Verbindungselements 15, die in Fig. 3e der Länge der Spitzen 59 entspricht, kann zwischen ca. 2 mm und 5 mm betragen, in diesem exemplarischen Beispiel beträgt die Länge der Spitzen 59 3 mm. Der Abstand 67 zwischen den Spitzen 59 kann zwischen ca. 1 mm und 2 mm betragen, in diesem exemplarischen Beispiel beträgt der Abstand 67 ca. 1,4 mm. Der Radius 69 zwischen den Spitzen 59 kann zwischen ca. 0,1 mm und 0,3 mm betragen, in diesem exemplarischen Beispiel beträgt der Radius ca. 0,2 mm.

Die Gesamtlänge 43 der drei rein exemplarischen Ausführungsformen beträgt in Fig. 3h 8 mm, in Fig. 3i 11 mm und in Fig. 3j 14 mm. Verschiedene Gesamtlängen 43 können vorteilhaft sein, wenn beispielsweise je nach vorliegender Kieferknochensubstanz und/oder je nach der Größe des Stützelements 13 zwischen verschiedenen Längen für den jeweiligen Einsatzzweck individuell ausgewählt werden kann.

**Fig. 3k** zeigt die Form des ersten Verbindungsabschnitt 25 analog zur Beschreibung zu Fig. 3a.

Die Länge des ersten Endabschnitts 38, die der Länge des ersten Verbindungsabschnitts 25 entspricht, kann rein exemplarisch 0,3 mm betragen.

Auf den konischen Bereich des ersten Verbindungsabschnitts 25 kann das Stützelement 13 aufgesteckt werden. Je nach der verwendeten Materialpaarung des Verbindungselements einerseits und des Stützelements 13 andererseits kann der Konuswinkel α derart gewählt werden, dass eine selbsthemmende Kegelpressverbindung entsteht. Der äußere Durchmesser 71 an der Konusbasis, von der aus der Konus sich zur Stirnseite hin verjüngt, kann in diesem exemplarischen Ausführungsbeispiel ca. 1,67 mm betragen.

Das Bezugszeichen 50 bezeichnet den Innendurchmesser des in dieser Ausführungsform optional hohl oder teilweise hohl ausgestalteten Stützelements 13.

**Fig. 4** zeigt das erfindungsgemäße zahntechnische Implantat 100 einer Ausführungsform im montierten Zustand mit Stützelement 13 und Verbindungselement 15.

**Fig. 4a** zeigt eine weitere Ausführungsform eines erfindungsgemäßen zahntechnischen Implantats 100. Die obere Darstellung der Fig. 4a ist eine perspektivische seitliche Ansicht, die untere Darstellung der Fig. 4a ist eine perspektivische Ansicht von schräg oben.

Die Form des Verbindungselements 15 ist ähnlich aufgebaut wie die Ausführungsform der Fig. 3e bis 3g. Das Stützelement 13 ist in dieser Ausführungsform in einer Draufsicht optional rund ausgeführt mit z. B. vier hier exemplarisch nach radial außen ausgerichteten Streben zwischen einem inneren scheibenförmigen Bereich und einem äußeren Ring des Stützelements 13. Das Vorstehen von Streben ist hier optional.

Die Befestigung des Stützelements 13 am Verbindungselement 15 erfolgt mittels dreier Stifte 91 oder einer anderen Anzahl von Stiften 91.

Die Stifte 91 sind optional integrale Bestandteile des Verbindungselements 15, also einstückig mit dem Verbindungselement 15 hergestellt.

Die Herstellung kann beispielsweise aus einem scheibenförmigen oder rohrförmigen Halbzeug als Ausgangsmaterial erfolgen, das mittels Laserschneiden bearbeitet wird und ggf. später in die in Fig. 4a gezeigte Zylinderform gebogen und optional entlang der Stoßränder in Längsrichtung miteinander verfügt wird, z. B. durch Laserschweißen.

Im nicht montierten Zustand des Stützelements 13 erstrecken sich die Stifte 91 in Längsrichtung optional mehr oder weniger parallel zur Längsachse des Stützelements 13.

Zur Montage des Stützelements 13 auf dem Verbindungselement 15 können diese Stifte 91 dann um ca. 90° (Grad) nach radial außen umgebogen werden, wie dies - als Ergebnis - in Fig. 4a dargestellt ist.

Zur exakten Positionierung des Stützelements 13 am Verbindungselement 15 während des Montierens kann die innere Scheibe des Stützelements 13 Einkerbungen zum Führen der Stifte 91 aufweisen, sie können in axialer Richtung verlaufen, als senkrecht zur radialen Richtung, in welcher die Streben sich erstrecken. Diese innere Scheibe liegt dann im montierten Zustand auf dem Verbindungselement 15 auf und wird mittels der umgebogenen Stifte 91 fixiert.

Die innere Scheibe kann eine zentrale Öffnung aufweisen. Sie kann dimensioniert sein, um mit ihrem Rand auf einem optional vorgesehenen Rand oder Absatz, wie er beispielsweise in Fig. 3k offenbart ist, aufgesetzt zu werden. Der in Fig. 3k gezeigte Konus - oder eine andere stirnseitige Ausgestaltung des Verbindungselements 15 kann somit in oder durch die zentrale Öffnung hinein- oder hindurchragen. Die zentrale Öffnung kann als Sacköffnung oder als Durchgangsöffnung ausgestaltet sein.

Die Höhenmarkierungen 55a sind, im Gegensatz zu den Höhenmarkierungen 55 der Fig. 3d und 3g, als kleine, runde Löcher oder Öffnungen (Durchgangsöffnungen oder Sacköffnungen) ausgeführt. Diese Höhenmarkierungen 55a können vorteilhaft dafür genutzt werden, um die Eindringtiefe des Verbindungselements 15 in den Kieferknochen zu bestimmen oder abzulesen. Die Höhenmarkierungen 55a können in Längsrichtung äquidistant oder nicht äquidistant angeordnet sein. Jede andere Ausgestaltung von Höhenmarkierungen als die in Fig. 4a gezeigte ist ebenfalls von der vorliegenden Erfindung umfasst.

Die in Fig. 4a dargestellte Ausführungsform des zahntechnischen Implantats 100 wird vorzugsweise im montierten Zustand in dem Kieferknochen fixiert. Im montierten Zustand ist das Stützelement 13 bereits am Verbindungselement 15 fixiert.

Das in Fig. 4a dargestellte zahntechnische Implantat 100 kann mit oder ohne Werkzeug im Kieferknochen, beispielsweise durch Einschlagen, fixiert werden.

Auch das in Fig. 4a dargestellte zahntechnische Implantat 100 kann ganz oder teilweise aus einem teilweise oder vollständig resorbierbaren Material hergestellt sein.

**Fig. 5** zeigt den Knochenaufbau am Kieferknochen 3 aus Fig. 1 mit dem erfindungsgemäßen zahntechnischen Implantat 100. Das Implantat 100 ist an der Implantationsstelle 41 im Kieferknochen 3 implantiert bzw. eingesteckt.

Das Stützelement 13 weist in dem in Fig. 5 dargestellten Implantationszustand des Implantats 100 eine der Mundhöhle des Patienten zugewandte Oberseite 45 und eine der Implantationsstelle 41 zugewandte Unterseite 47 auf.

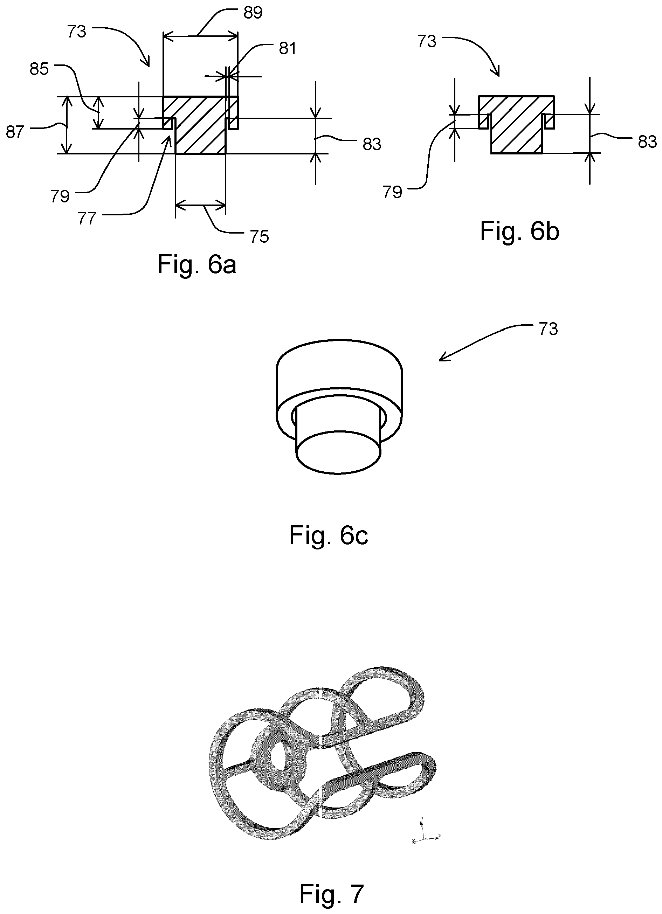

**Fig. 6a-c** zeigen ein beispielhaftes Werkzeug 73 zum Einschlagen des Verbindungselements 15 in den Kieferknochen in zwei verschiedenen Ausführungsformen und in unterschiedlichen Ansichten.

**Fig. 6a** zeigt das Werkzeug 73 in einer Halbschnittdarstellung in einer ersten Ausführungsform. Die nachfolgenden Zahlenangaben sind rein exemplarisch und können insbesondere für verschiedenen Verbindungselemente 15 hiervon abweichen.

Der Zapfendurchmesser 75 kann derart gewählt werden, dass das Werkzeug 73 möglichst spielfrei auf den ersten Verbindungsabschnitt 25 des Verbindungselements 15 aufgesetzt oder, sollte dieses zumindest in seinem oberen Abschnitt als Hohlkörper ausgestaltet sein, in den Innenquerschnitt oder dessen Innenlumen eingeschoben werden kann (siehe Fig. 3k). Bei einem exemplarischen Außendurchmesser 49 von 1,8 mm und einer Wandstärke (im nichtkonischen Abschnitt) von 0,16 mm beträgt der Innendurchmesser 1,48 mm. Dieser Innendurchmesser, welcher in Fig. 3k mit dem Bezugszeichen 50 versehen ist, entspricht somit dem Zapfendurchmesser 75 des Werkzeugs 73, gegebenenfalls mit einer auszuwählenden Passung.

Die Ausführungsform in Fig. 6a ist derart gewählt, dass der erste Verbindungsabschnitt 25 des Verbindungselements 15 (siehe Fig. 3k) stirnseitig auf dem Werkzeug 73 in einem optional umlaufenden Spaltabschnitt 77 und vorzugsweise auf dessen Grund (in Fig. 6a ganz oben) aufsetzt. Anders ausgedrückt ist die Ausführungsform des Werkzeugs 73 in Fig. 6a ein Werkzeug 73 mit einer Auflage zum Anlegen des Werkzeugs 73 auf den Konus des Verbindungselements 15. Entsprechend dem exemplarischen Zahlenbeispiel aus Fig. 3k beträgt die Höhe 79 des Spaltabschnitts 77 0,3 mm.

Die Spaltbreite 81 entspricht nach dem exemplarischen Zahlenbeispiel aus Fig. 3k 0,95 mm und wird aus der exemplarischen Wandstärke von 0,16 mm abzüglich der Konusbreite (bezogen auf den äußeren Durchmesser 71 an der Konusbasis) von 0,65 mm errechnet. Die übrigen Maße können entsprechend angepasst werden, es ergibt sich also rein exemplarisch eine Zapfenlänge 83 von 1,1 mm, eine obere Teillänge 85 (Länge vor einer oberen Stirnseite bis zu einer unteren Stirnseite des Spaltabschnitts) des Werkzeugs 73 von 1 mm, eine Gesamtlänge 87 des Werkzeugs 73 von oberer Stirnseite zu unterer Stirnseite von 1,7 mm und ein Außendurchmesser 89 des Werkzeugs 73 von 2,2 mm. Auf die obere Stirnseite des Werkzeugs 73 mit dem Außendurchmesser 89 kann die Kraft zum Einschlagen des Verbindungselements 15 in den Kieferknochen ausgeübt werden.

Dabei ist bei der Ausführungsform der Fig. 6a zu beachten, dass die Stirnseite des Konus des Verbindungselements 15 auf der endseitigen Stirnfläche des Spaltabschnitts 77 (also auf dem Spaltgrund) auf dem Werkzeug 73 gut aufliegt. Eine Verformung des Konus des Verbindungselements 15 bei dessen Einschlagen kann hierdurch vorteilhaft vermieden werden, da sonst die Gefahr besteht, dass das Stützelement 13 nach Einschlagen nicht mehr passgenau auf den Konus des Verbindungselements 15 aufgesetzt werden kann.

Als Material für das Werkzeug kann beispielsweise ein Metall oder ein Kunststoff verwendet werden, beispielsweise ein Polyetheretherketon (abgekürzt PEEK).

**Fig. 6b** zeigt das Werkzeug 73 in einer zweiten Ausführungsform. In dieser exemplarischen Ausführungsform ist die Höhe 79 des Spaltabschnitts 77 derart gewählt, dass die Stirnfläche des Konus des Verbindungselements 15 nicht auf der endseitigen Stirnfläche des Spaltabschnitts 77 (also auf dem Spaltgrund) des Werkzeugs 73 beim Einschlagen aufliegt. Vielmehr liegt dabei die in Fig. 6c von unten zu sehende freie Ringfläche des den Spaltabtschnitt 77 konzentrisch umgebenden Zylinderabschnitts auf dem in Fig. 3k als horizontaler Ring zu erkennenden Absatz der Außenwandung des Verbindungselements 15. Daher kann die Höhe 79 des Spaltabschnitts 77 rein exemplarisch beispielsweise 0,4 mm betragen. Bei den gleichen übrigen äußeren Abmaßen entsprechend dem Beispiel aus Fig. 6a beträgt dann die exemplarische Zapfenlänge 83 1,1 mm.

Aufgrund der Tatsache, dass die Stirnfläche des Konus des Verbindungselements 15 in dieser Ausführungsform beim Einschlagen nicht auf der endseitigen Stirnfläche des Spaltabschnitts 77 (also auf dem Spaltgrund) aufliegt, sondern der Zylinderabschnitt auf dem Absatz zu liegen kommt, kann vorteilhaft eine mechanische Beschädigung des Konus bei Einschlagen vermieden werden.

**Fig. 6c** zeigt eine perspektivische Ansicht des Werkzeugs 73 entsprechend den Ausführungsformen aus Fig. 6a und Fig. 6b mit Blick von unten auf die untere Stirnseite sowie mit Blick von unten in den Spaltabschnitt 77 hinein.

**Fig. 7** zeigt eine Fertigungsvariante für das Stützelement 13. In dieser Ausführungsform kann das Stützelement 13 aus einem rohrförmigen Rohmaterial hergestellt werden. Das Rohmaterial kann beispielsweise das gleiche Rohmaterial sein, wie es zur Herstellung des Verbindungselements 15 nach den Ausführungsformen in Fig. 3a bis 3k verwendet werden kann. Der rein exemplarische Außendurchmesser 49 des Verbindungselements 15 wurde in diesen Figurenbeschreibungen mit 1,8 mm angegeben. Entsprechend der beschriebenen Abwicklung in Fig. 3h entspricht dies einer Länge von ca. 5,7 mm. Bei einer exemplarischen Breite 33 des Stützelements 13 von 5 mm reicht dies zur Fertigung des Stützelements 13 aus. Die Fertigung kann beispielsweise mittels Laserschneidens erfolgen. Das grundsätzliche Verfahren für ein resorbierbares Material wie beispielsweise eine Magnesiumlegierung ist aus der Fertigung für Stents aus der medizintechnischen Anwendung bekannt. Allerdings muss das Stützelement nach der Fertigung plastisch verformt bzw. aufgeweitet werden.

**Bezugszeichenliste**

| | |
|---|---|
| 100 | zahntechnisches Implantat |
| $\alpha$ | Konuswinkel des ersten Verbindungsabschnitts |
| $\mu_a$ | Reibungskoeffizient in Axialrichtung |
| 1 | Knochenersatzmaterial; Granulat |
| 3 | Kieferknochen |
| 5 | Zahn |
| 7 | Schraube; Platzhalter |
| 9 | Abdeckmembran; Abdeckfolie; Deckelement |
| 11 | Pfeilrichtung zum Bewegen der Abdeckmembran |

| | |
|---|---|
| 13 | Stützelement |
| 15 | Verbindungselement |
| 17 | Querstrebe |
| 19 | Längsstrebe |
| 21 | radiale Strebe |
| 23 | Einstecköffnung; zweiter Verbindungsabschnitt |
| 25 | Einsteckstift; erster Verbindungsabschnitt |
| 31 | Länge des Stützelements |
| 32 | Dicke der Querstreben und/oder der Längsstreben |
| 33 | Breite des Stützelements |
| 34 | Dicke der äußeren, umlaufenden Streben |
| 35 | äußerer Durchmesser des runden Stützelements |
| 36 | Höhe des Stützelements |
| 37 | erster Endabschnitt des Verbindungselements |
| 38 | Länge des ersten Endabschnitts |
| 39 | zweiter Endabschnitt des Verbindungselements |
| 41 | Implantationsstelle |
| 43 | Gesamtlänge des Verbindungselements |
| 45 | Oberseite des Stützelements |
| 47 | Unterseite des Stützelements |
| 49 | Außendurchmesser des Verbindungselements |
| 50 | Innendurchmesser |
| 51 | Schlitz |
| 53 | Bohrung |
| 55,55a | Höhenmarkierungen des Verbindungselements |
| 57 | Pfeilrichtung |
| 59 | Spitzen |
| 61 | obere Teillänge des Verbindungselements |
| 63 | untere Teillänge des Verbindungselements |
| 65 | Länge der Abwicklung; Umfang |
| 67 | Abstand zwischen den Spitzen |
| 69 | Radius zwischen den Spitzen |
| 71 | äußerer Durchmesser an der Konusbasis |
| 73 | Werkzeug zum Einschlagen des Verbindungselements |
| 75 | Zapfendurchmesser |
| 77 | Spaltabschnitt |
| 79 | Höhe des Spaltabschnitts |
| 81 | Spaltbreite |
| 83 | Zapfenlänge |
| 85 | obere Teillänge des Werkzeugs |
| 87 | Gesamtlänge des Werkzeugs |
| 89 | Außendurchmesser des Werkzeugs |
| 91 | Stift |

**Patentansprüche**

1. Zahntechnisches Implantat (100) zum Abstützen, insbesondere mittels einer Stützstruktur, eines Deckelements (9), aufweisend

    - ein Verbindungselement (15) mit

       - einem ersten Endabschnitt (37) und mit
       - einem zweiten Endabschnitt (39) zum Verbinden des Implantats (100) mit einem Kieferknochen (3); und

    - ein erstes Stützelement (13) zum Abstützen eines Deckelements (9) nach Implantation des Implantats (100) im Kieferknochen (3);
    wobei der erste Endabschnitt (37) als ein erster Verbindungsabschnitt (25) ausgestaltet ist oder einen solchen aufweist;

wobei das erste Stützelement (13) einen zweiten Verbindungsabschnitt (23) aufweist;

wobei der erste Verbindungsabschnitt (25) und der zweite Verbindungsabschnitt (23) ausgestaltet sind, um untereinander oder miteinander verbindbar zu sein;

wobei der zweite Endabschnitt (39) als ein Implantationsabschnitt zum vorübergehenden Implantieren des Verbindungselements (15) an oder in der Implantationsstelle (41) des Kieferknochens (3) ausgestaltet ist oder einen solchen aufweist.

2. Zahntechnisches Implantat (100) nach Anspruch 1, wobei der erste und der zweite Verbindungsabschnitt (25, 23) einander entsprechen, wobei der erste Verbindungsabschnitt (25) oder der zweite Verbindungsabschnitt (23) vorzugsweise als Einstecköffnung (23) ausgestaltet ist, und wobei der andere dieser beiden Verbindungsabschnitte (25, 23) als Einsteckstift (25) ausgestaltet ist.

3. Zahntechnisches Implantat (100) nach Anspruch 1 oder Anspruch 2, wobei der zweite Endabschnitt (39) als zulaufender oder sich verjüngender Abschnitt ausgestaltet ist.

4. Zahntechnisches Implantat (100) nach einem der vorangegangenen Ansprüche, wobei der erste Verbindungsabschnitt (25) und der zweite Verbindungsabschnitt (23) zur lösbaren Verbindung miteinander ausgestaltet sind.

5. Zahntechnisches Implantat (100) nach einem der vorangegangenen Ansprüche, wobei das erste Stützelement (13) eine gitterförmige Stützstruktur ist oder eine solche aufweist.

6. Zahntechnisches Implantat (100) nach einem der vorangegangenen Ansprüche, wobei das erste Stützelement (13) eine im Implantationszustand des Implantats (100) der Mundhöhle des Patienten zugewandte Oberseite (45) und eine der Implantationsstelle (41) zugewandte Unterseite (47) aufweist, wobei der zweite Verbindungsabschnitt (23) ausgestaltet ist, um den ersten Verbindungsabschnitt (25) von der Unterseite (47) her aufzunehmen.

7. Zahntechnisches Implantat (100) nach einem der vorangegangenen Ansprüche, wobei das Verbindungselement (15) keinen Gewindeabschnitt und/ oder keinen Schraubenkopf aufweist.

8. Zahntechnisches Implantat (100) nach einem der vorangegangenen Ansprüche, wobei das Verbindungselement (15) und/ oder das erste Stützelement (13) aus einem Metall, einem Kunststoff und/ oder einem Verbundmaterial hergestellt ist oder ein solches Material aufweist.

9. Zahntechnisches Implantat (100) nach einem der vorangegangenen Ansprüche, wobei das Verbindungselement (15) und/ oder das erste Stützelement (13) ganz oder teilweise aus einem resorbierbaren Material hergestellt ist oder ein solches Material aufweist.

10. Set, umfassend ein zahntechnisches Implantat (100) nach einem der vorangegangenen Ansprüche, ferner aufweisend

- wenigstens ein weiteres Stützelement (13), wobei sich das erste Stützelement (13) und das zweite Stützelement (13) in wenigstens einem geometrischen Merkmal unterscheiden, und/oder
- ein Zahnimplantat.

11. Set nach Anspruch 10, wobei das geometrische Merkmal die Form, eine Abmessung, die Fläche und/ oder die Anordnung von Stützstrukturelementen umfasst.

12. Werkzeug (73) zum Einbringen eines Verbindungselements (15) eines zahntechnischen Implantats (100) nach einem der Ansprüche 1 bis 9 in einen Knochen, wobei das Werkzeug (73) einen Spaltabschnitt (77) zum Aufnehmen eines Endbereichs des Verbindungselements (15) aufweist.


## Claims

1. A dental prosthetic implant (100) for supporting, in particular by means of a support structure, a covering element (9), comprising:

- a connecting element (15) having:

- a first end portion (37) and
- a second end portion (39) for connecting the implant (100) to a jawbone (3); and

- a first support element (13) for supporting a covering element (9) after implantation of the implant (100) in the jawbone (3);

wherein the first end portion (37) is designed as a first connecting portion (25) or comprises such one;
wherein the first support element (13) comprises a second connecting portion (23);
wherein the first connecting portion (25) and the second connecting portion (23) are designed to be connectable to each other or with each other;
wherein the second end portion (39) is designed as an implantation section or comprises such one for temporarily implanting the connecting element (15) at or in the implantation site (41) of the jawbone (3).

2. The dental prosthetic implant (100) according to claim 1, wherein the first and the second connecting portions (25, 23) correspond to each other, the first connecting portion (25) or the second connecting portion (23) being preferably designed as an insertion opening (23), and the other of these two connecting portions (25, 23) being designed as an insertion pin (25).

3. The dental prosthetic implant (100) according to claim 1 or 2, wherein the second end portion (39) is designed as a converging or tapering portion.

4. The dental prosthetic implant (100) according to any one of the preceding claims, wherein the first connecting portion (25) and the second connecting portion (23) are designed for releasable connection with each other.

5. The dental prosthetic implant (100) according to any one of the preceding claims, wherein the first support element (13) is a grid-shaped support structure or comprises such one.

6. The dental prosthetic implant (100) according to any one of the preceding claims, wherein the first support element (13) comprises an upper side (45) facing the oral cavity of the patient in the implantation state of the implant (100) and a lower side (47) facing the implantation site (41),
the second connecting portion (23) being designed to receive the first connecting portion (25) from the lower side (47).

7. The dental prosthetic implant (100) according to any one of the preceding claims, wherein the connecting element (15) does not comprise a threaded section and/or a screw head.

8. The dental prosthetic implant (100) according to any one of the preceding claims, wherein the connecting element (15) and/or the first support element (13) is/are made of a metal, a plastic and/or a composite material or comprise(s) such a material.

9. The dental prosthetic implant (100) according to any one of the preceding claims, wherein the connecting element (15) and/or the first support element (13) is/are made entirely or partially of a resorbable material or comprise(s) such a material.

10. A set comprising a dental prosthetic implant (100) according to any one of the preceding claims, further comprising:

- at least one further support element (13), wherein the first support element (13) and the second support element (13) differ in at least one geometric feature, and/or
- a dental implant.

11. The set according to claim 10, wherein the geometric feature comprises the form, one dimension, the area, and/or the arrangement of support structure elements.

12. A tool (73) for inserting a connecting element (15) of a dental prosthetic implant (100) according to any one of claims 1 to 9, into a bone, wherein the tool (73) comprises a slot section (77) for receiving an end region of the connecting element (15).

**Revendications**

1. Un implant dentaire prothétique (100) destiné à supporter, notamment au moyen d'une structure de support, un élément de recouvrement (9), comprenant :

   - un élément de liaison (15) avec :

     - une première extrémité (37) ainsi
     - qu'une seconde extrémité (39) destinée à relier l'implant (100) à un os de la mâchoire (3); et

   - un premier élément de support (13) destiné à supporter un élément de recouvrement (9) après implantation de l'implant (100) dans l'os de la mâchoire (3);

     où la première extrémité (37) est conçue comme une première section de liaison (25) ou en possède une;
     où le premier élément de support (13) possède une seconde section de liaison (23);
     où la première section de liaison (25) ainsi que la seconde section de liaison (23) sont conçues pour pouvoir être reliées entre elles ou l'une à l'autre;
     où la seconde extrémité (39) est conçue comme une partie d'implantation, ou en possède une, destinée à l'implantation temporaire de l'élément de liaison (15) au ou dans le site d'implantation (41) de l'os de la mâchoire (3).

2. L'implant dentaire prothétique (100) selon la première revendication, où la première et la seconde section de liaison (25, 23) correspondent l'une à l'autre, la première section de liaison (25) ou la seconde section de liaison (23) étant conçue de préférence comme une ouverture d'insertion (23), et l'autre de ces deux sections de liaison (25, 23) étant conçue comme une broche d'insertion (25).

3. L'implant dentaire prothétique (100) selon la revendication 1 ou 2, où la seconde extrémité (39) est conçue comme une partie convergente ou effilée.

4. L'implant dentaire prothétique (100) selon l'une quelconque des revendications précédentes, où la première section de liaison (25) ainsi que la seconde section de liaison (23) sont conçues pour être reliées entre elles de manière amovible.

5. L'implant dentaire prothétique (100) selon l'une quelconque des revendications précédentes, où le premier élément de support (13) est une structure de support en forme de grille ou comprend une telle structure.

6. L'implant dentaire prothétique (100) selon l'une quelconque des revendications précédentes, où le premier élément de support (13) possède une face supérieure (45) orientée vers la cavité buccale du patient dans l'état d'implantation de l'implant (100) ainsi qu'une face inférieure (47) orientée vers le site d'implantation (41), la seconde section de liaison (23) étant conçue pour recevoir la première section de liaison (25) par la face inférieure (47).

7. L'implant dentaire prothétique (100) selon l'une quelconque des revendications précédentes, où l'élément de liaison (15) ne possède pas de partie filetée et/ou de tête de vis.

8. L'implant dentaire prothétique (100) selon l'une quelconque des revendications précédentes, où l'élément de liaison (15) et/ou le premier élément de support (13) est/sont fabriqué (s) à partir d'un métal, d'un plastique et/ou d'un matériau composite ou comporte(nt) un tel matériau.

9. L'implant dentaire prothétique (100) selon l'une quelconque des revendications précédentes, où l'élément de liaison (15) et/ou le premier élément de support (13) est/sont entièrement ou partiellement fabriqué(s) à partir d'un matériau résorbable ou comporte(nt) un tel matériau.

10. Un ensemble comprenant un implant dentaire prothétique (100) selon l'une quelconque des revendications précédentes, comprenant en outre :

    - au moins un élément de support supplémentaire (13), le premier élément de support (13) ainsi que le second élément de support (13) différant par au moins une caractéristique géométrique, et/ou
    - un implant dentaire.

**11.** L'ensemble selon la revendication 10, où la caractéristique géométrique comprend la forme, une dimension, la surface et/ou l'agencement des éléments de structure de support.

**12.** Un outil (73) pour l'insertion d'un élément de liaison (15) d'un implant dentaire prothétique (100), selon l'une quelconque des revendications 1 à 9, dans un os,
où l'outil (73) comporte une partie fendue (77) destinée à recevoir une zone d'extrémité de l'élément de liaison (15).

Fig. 1

Fig. 2a

Fig. 2b

Fig. 2c

Fig. 2d

Fig. 3a

Fig. 3b

Fig. 3c

Fig. 3d

Fig. 3e

Fig. 3f

Fig. 3g

Fig. 3h

Fig. 3i

Fig. 3j

Fig. 3k

Fig. 4

Fig. 4a

Fig. 5

Fig. 6a

Fig. 6b

Fig. 6c

Fig. 7

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102016000236 A1
- US 5769637 A
- KR 101231581 B1
- US 2005192675 A1
- EP 2744531 B1
- EP 2744532 B1